# FASCICULE DE BREVET EUROPEEN

(11) **EP 0 705 596 B1**
(45) Date de publication et mention de la délivrance du brevet: **27.11.1996**
(21) Numéro de dépôt: 95401996.4
(22) Date de dépôt: 01.09.1995
(51) Int. Cl.: A61K 7/09

(54) **Procédé pour la déformation non permanente des fibres kératiniques humaines**
Verfahren zur nicht-dauernden Verformung menschlicher keratinischer Fasern
Process for non-permanent deforming of human keratinic fibers

(30) Priorité: 04.10.1994 FR 9411852
(43) Date de publication de la demande: 10.04.1996
(73) Titulaire: L'OREAL, 75008 Paris (FR)
(72) Inventeur: Dupuis, Christine, F-75018 Paris (FR)
(74) Mandataire: Tezier Herman, Béatrice

(56) Documents cités:
- EP-A- 0 129 807
- FR-A- 2 273 492

## Description

La présente invention concerne un procédé amélioré de traitement des fibres kératiniques humaines, en particulier des cheveux, en vue notamment d'obtenir une déformation et/ou une mise en forme non permanente de ces dernières, en particulier sous la forme d'une mise en plis, ledit procédé étant notamment utilisable dans le domaine des salons de coiffure, de beauté, de cosmétique et analogues, professionnels. Plus précisément encore, elle concerne un procédé mettant en oeuvre de la vapeur d'eau et des substances traitantes particulières.

On sait que l'on désigne en coiffure sous le terme de "mise en plis" l'opération simple qui consiste à donner aux cheveux une forme non définitive et temporaire (généralement ondulée telle que boucles, frisures et autres) qui disparait instantanément lorsque les cheveux sont à nouveau mouillés, en particulier lorsque ceux-ci sont soumis à l'action de lavages à l'eau ou par shampooings, et ceci par opposition à une opération de déformation dite permanente, au cours de laquelle de véritables traitements et/ou transformations chimiques (réduction/oxydation) doivent être mis en oeuvre sur les fibres kératiniques, la forme finale imposée aux cheveux ne devenant dans ce cas plus du tout (ou que très peu) sensible aux agents extérieurs susmentionnées.

La technique la plus usuelle pour réaliser une mise en plis (ou déformation non permanente) des cheveux consiste à mettre tout d'abord sous tension (avec des supports classiques de type bigoudis, rouleaux de mise en plis et autres) des cheveux préalablement mouillés ou encore humides, puis à sécher les cheveux ainsi mis sous tension sous un casque de coiffure chauffant à une température comprise entre 30°C et 60°C pendant un temps qui peut varier de 20 à 60 mn selon la masse des cheveux à sécher, à ensuite retirer des cheveux ainsi séchés les moyens de mises sous tension utilisés précédemment et enfin à donner un coup de peigne de finition pour obtenir la coiffure avec la forme finale recherchée. Un autre procédé, moins usité, consiste à utiliser la technique ancienne dite du fer à friser ou du fer à coiffer (mèche de cheveux humide enroulée autour d'un mandrin métallique et portée par celui-ci à plus de 100°C pendant au moins 20 secondes); cette dernière technique est aujourd'hui peu pratiquée chez les coiffeurs professionnels car elle procure des résultats jugés dans l'ensemble peu satisfaisants et irréguliers en raison du fait, notamment, que les cheveux sont soumis à des températures très différentes selon qu'ils sont au contact même ou au contraire assez éloignés du mandrin chauffant.

Dans le document FR-A- 2 273 492, on a déjà proposé de faire appel à des traitements à la vapeur d'eau surchauffée dans le but, entre autres, d'améliorer la qualité et/ou les rendements des mises en plis sur cheveux. Bien que cette technique permette effectivement, par rapport aux procédés classiques, d'améliorer tout de suite certaines caractéristiques de la mise en plis, et en particulier le rendement de la mise en plis (ou degré de frisure), ces améliorations restent néanmoins d'une durabilité (ou tenue) dans le temps limitée, puisque disparaissant généralement dans les quelques jours qui suivent le traitement sous l'influence du mouvement, des peignages et brossage répétés et de l'écrasement de la chevelure durant le sommeil. Par ailleurs, indépendamment de l'aspect rétention du degré de frisure évoqué précédemment, il serait bien évidemment intéressant de pouvoir disposer de cheveux bouclés présentant un degré de frisure initial (c.à.d. juste après traitement de mise en plis) et une tenue de frisure encore améliorés par rapport à ce qui est connu à ce jour, avec ou sans traitement à la vapeur d'eau.

La présente invention a notamment pour but de résoudre les problèmes ci-dessus.

Plus précisément encore, la présente invention a pour but de proposer un nouveau procédé de traitement convenant à la déformation non permanente des fibres kératiniques humaines, en particulier des cheveux, qui permette notamment d'obtenir des frisures de haute qualité.

Elle a également pour but de proposer un procédé tel que ci-dessus qui permette en outre de conserver ces frisures de manière durable dans le temps (rétention), même après avoir subi des déformations.

Or, à la suite d'importantes recherches menées sur la question, il a maintenant été trouvé par la Demanderesse, et ceci de façon tout à fait inattendue et surprenante, que ces buts, et d'autres, pouvaient être atteints en mettant en oeuvre, selon certaines conditions particulières, de la vapeur d'eau sur des fibres kératiniques humaines préalablement traitées avec des protéines et/ou des dérivés protéiniques. Cette découverte est à la base de la présente invention.

Ainsi, il est maintenant proposé selon la présente invention un nouveau procédé de traitement convenant à la déformation et/ou la mise en forme, et ceci de manière non permanente, des fibres kératiniques humaines, et en particulier des cheveux, ledit procédé étant caractérisé par le fait qu'il consiste (i) à mettre en contact un gaz contenant de la vapeur d'eau et dont la température est d'au moins 75°C, avec des fibres kératiniques humaines maintenues sous tension mécanique (rouleaux, bigoudis ou autres) et sur lesquelles on a appliqué une composition contenant au moins une protéine et/ou un dérivé protéinique, (ii) à refroidir les fibres ainsi traitées et enfin (iii) à supprimer la tension mécanique qui était appliquée sur les fibres.

Bien que l'exposé qui suit s'articule essentiellement autour du cas particulier du traitement des cheveux, on notera ici que le procédé selon l'invention est applicable à toute matière kératinique humaine en général, notamment cils, moustaches, poils et autres.

Le gaz chaud traitant contient de préférence au moins 1% en volume de vapeur d'eau.

En plus de la vapeur d'eau, le gaz vecteur (ou support) peut contenir de la vapeur de solvant, ainsi que des gaz tels que l'oxygène ou l'azote, des mélanges de gaz tels que l'air ou bien encore d'autres composés vaporisables.

A titre de solvants qui peuvent être avantageusement utilisés pour la production de vapeur (mélanges eau-solvant), on fait plus particulièrement appel aux solvants organiques cosmétiquement acceptables, comme par exemple des alcools tels que l'éthanol, l'isopropanol, l'alcool benzylique, l'alcool phényléthylique ou des glycols ou éthers de glycol tels que par exemple l'éthylène glycol et ses éthers monométhylique, monoéthylique et monobutylique, le propylène glycol, le butylène glycol, le dipropylèneglycol, ainsi que les alkyléthers comme le monobutyléther du diéthylèneglycol.

Selon la présente invention, le gaz est constitué de préférence soit uniquement ou essentiellement de vapeur d'eau, soit d'un mélange d'eau et d'air.

La température du gaz est de préférence supérieure ou égale à 85°C, et est plus particulièrement comprise entre 85°C et 150°C environ.

De manière préférée, le temps de contact entre le gaz chaud traitant et la fibre est bref et n' excède avantageusement pas 2 minutes. De préférence, le gaz est mis en contact avec la fibre pendant une durée allant de 0,01 seconde à 30 secondes, et encore plus préférentiellement de 1 seconde à 10 secondes. Bien entendu, l'application du gaz peut être répétée plusieurs fois sur la même fibre, chaque opération se faisant selon une durée telle qu'indiquée ci-dessus.

Un mode préféré de réalisation du procédé selon l'invention consiste à appliquer tout d'abord sur les cheveux une composition constituée pour partie ou totalement de protéines et/ou de dérivés protéiniques, cette application pouvant être réalisée avant, pendant ou après l'habituelle opération de mise sous tension des mèches de cheveux sous une forme correspondant à la forme finale désirée pour ces derniers (boucles par exemple), cette opération pouvant elle-même être mise en oeuvre par tout moyen, mécanique notamment, approprié et connu en soi pour maintenir sous tension des cheveux, tels que par exemple corps tubulaires, rouleaux, bigoudis et autres, puis à soumettre ces mèches ainsi imprégnées de protéines ou de dérivés protéiniques à l'action brève de la vapeur d'eau selon les conditions mentionnées ci-avant, l'action de la vapeur d'eau étant de préférence effectuée après séchage des cheveux imprégnés, puis à refroidir, de préférence rapidement, les mèches ainsi traitées à la vapeur, par exemple en envoyant sur ou à travers celles-ci un courant d'air à température ambiante et/ou en aspirant un courant d'air ambiant à travers les mèches enroulées, et enfin, de préférence après séchage des cheveux, à retirer des cheveux les moyens mécaniques qui maintenaient ces derniers sous tension et dans la forme désirée tout au long du traitement, ce par quoi l'on obtient finalement des mèches ou une chevelure présentant par exemple de belles boucles régulières et douces.

La production d'un gaz chaud comprenant de la vapeur d'eau peut se faire à l'aide de tout appareil connu en soi et prévu à cet effet. Toutefois, selon la présente invention, on utilise de préférence un appareil tel que celui décrit dans la demande de brevet français FR-A- 2 273 492, ou tout autre appareil équivalent, qui convient en effet dans le cas présent particulièrement bien (traitement ponctuel, régulier et homogène des fibres, sans risque de surchauffe, avec post-traitement de refroidissement intégré).

Les protéines utilisables selon l'invention peuvent être issues de matières d'origine animale ou végétale.

A titre d'exemples de protéines d'origine animale convenant à la mise en oeuvre du procédé selon l'invention, on peut notamment citer :
- la kératine, qui peut provenir des cheveux, de la laine, de la corne, des poils, des soies, des écailles et des plumes, notamment par broyage,
- l'élastine,
- le collagène, qui peut être obtenu à partir de peau de poisson ou de volaille. On peut citer à titre d'exemple les collagènes vendus sous les dénominations "COLLAGENE NATIF MARIN" par la société SCHMITT JOURDAN, "LIPOCOLLAGENE" par la société SILAB, ou "COLLAGENE" par la société MATHE,
- les protéines extraites du lait telles que la lactoferrine, la caséine, le caséinate de sodium, de magnésium ou de calcium, les protéines du babeurre, les protéines du lactosérum dont l'α-lactalbumine, la β-lactoglobuline et les immunoglobulines,
- l'albumine de blanc d'oeuf.

A titre d'exemples de protéines d'origine végétale convenant à la mise en oeuvre du procédé selon l'invention, on peut notamment citer les protéines extraites du blé, de germes de blé, de l'avoine, de l'orge, du maïs, du riz, du soja, de fèves, de graines de coton, de graines de lupin, de pommes de terre, de noyaux d'abricots.

Des protéines extraites du blé sont notamment vendues sous la dénomination "SUPER EXTRAIT PLACENTAIRE DE BLE" par la société COSMETOCHEM.

Des protéines extraites de germes de blé sont notamment vendues sous les dénominations "BIOGERM 1066" par la société CFPA, "TENSEUR VEGETAL" par la société JAN DEKKER.

Des protéines extraites de l'avoine sont notamment vendues sous la dénomination "THERMAR 63 (BLE)" par la société IAM.

Des protéines extraites de l'orge sont notamment vendues sous la dénomination "FARINE D'ORGE" par la société CEREALES VENTOUX.

Des protéines extraites du soja sont notamment vendues sous les dénominations "CENPRO 70" par la société JAN DEKKER, "SOYBEAN PROTEIN ISOLATE" par la société NESTLE.

Des protéines extraites de fèves sont notamment vendues sous la dénomination "CONCENTRAT 65" par la société GEMEF.

Des protéines extraites de graines de lupin sont notamment vendues sous la dénomination "CONCENTRAT DE LUPIN" par la société USSI.

Des protéines extraites de noyaux d'abricots sont notamment vendues sous les dénominations "ABRILOR 4" par la société NESTLE.

On peut également utiliser, pour la mise en oeuvre du procédé selon l'invention, des dérivés de protéines. Par dérivés de protéines, on entend des protéines qui ont été soumises à des opérations chimiques classiques telles que l'hydrolyse, l'oxydation, le greffage ou la quaternisation dans le but soit de modifier certaines de leurs propriétés physiques soit d'introduire des fonctionnalités nouvelles aux extrémités de la chaîne peptidique ou sur la chaîne peptidique (modification des propriétés chimiques) comme par exemple des groupements anioniques (sulfates, sulfonates, phosphates, carboxylates), cationiques (ammoniums quaternaires) ou neutres (acétyles et hydroxyalkyles par exemple).

Les dérivés de protéines sont de préférence des hydrolysats de protéines qui peuvent être quaternisés.

Comme exemple d'hydrolysats de protéines utilisables selon l'invention, on peut notamment citer :
- les hydrolysats de kératine de plumes tels que ceux vendus sous la dénomination "MONTEINE KPL" par la société SEPPIC,
- les hydrolysats de kératine de laine tels que ceux vendus sous les dénominations "CROTEINE WKP" par la société CRODA, "PROMOIS WK-H" par la société SEIWA KASEI, "MONTEINE WK-HP" par la société SEPPIC,
- les hydrolysats de kératine de sabots de bovins tels que ceux vendus sous les dénominations "KERASOL", "KERASOL OR PR 2047" par la société CRODA,
- les hydrolysats d'élastine tels que ceux vendus sous les dénominations "ELASTINE MARINE" par la société LIB ou "NUTRILAN ELASTINE E 20" par la société HENKEL,
- les hydrolysats de collagène tels que ceux vendus sous les dénominations "CROTEINE SPO" par la société CRODA ou "PROMOIS W-32 R" par la société SEIWA KASEI,
- les hydrolysats de caséine tels que ceux vendus sous les dénominations "HYDROLACTIN 2500" par la société CRODA, "PEPTIDES N3" par la société ARMOR PROTEINES, "PROMOIS MILK P" par la société SEIWA KASEI,
- les hydrolysats de protéines de lactosérum tels que ceux vendus sous la dénomination "PEPTIDES 80" par la société ARMOR PROTEINES,
- les hydrolysats de protéines de blanc d'oeuf tels que ceux vendus sous la dénomination "OVAMINE" par la société SCHMITT JOURDAN,
- les hydrolysats de protéine de soie tels que ceux vendus sous la dénomination "PROMOIS SILK 1000 P" par la société SEIWA KASEI,
- les hydrolysats d'algues tels que ceux vendus sous la dénomination "PROTALG" par la société BIOPREX,
- les hydrolysats de protéines de blé tels que ceux vendus sous les dénominations "CROPEPSOL W" et "TRITISOL" par la société CRODA, "PEPTEIN VGW" par la société LASERSON, "HYDROTRITICUM 2000 POWDER" par la société CRODA, "TENSINE" et "SITOLINE" par la société SILAB, "PROMOIS WG" par la société SEIWA KASEI,
- les hydrolysats de protéines de soja tels que ceux vendus sous les dénominations "PEPTEIN VGS" par la société HORMEL, "PEPTIDES DE SOJA" par la société SOLABIA, "HYDROSOY" par la société CRODA, "FXP-W-0003" par la société PROTEIN TECHNOLOGIES,
- les hydrolysats de protéines de lupin blanc tels que ceux vendus sous la dénomination "PEPTIDES DE LUPIN" par la société SILAB.

Comme exemple d'hydrolysats de protéines quaternisés, on peut citer :
- les hydrolysats de kératine de laine portant des groupements N-hydroxypropyl diméthyl cocoylamidopropyl ammonium tels que ceux vendus sous la dénomination "MONTEINE LKA" par la société SEPPIC, ou des groupements cocoyl diméthyl N-hydroxypropyl ammonium tels que ceux vendus sous les dénominations "CROQUAT WKP" par la société CRODA ou "PROMOIS WK-HCAQ" de la société SEIWA KASEI, ou des groupements stéaryl diméthyl N-hydroxypropyl ammonium tels que ceux vendus sous la dénomination "PROMOIS WK-HSAQ" par la société SEIWA KASEI, ou des groupements lauryl diméthyl N-hydroxypropyl ammonium tels que ceux vendus sous la dénomination "PROMOIS WK-HLAQ" par la société SEIWA KASEI, ou des groupements triméthyl N-hydroxypropyl ammonium tels que ceux vendus sous la dénomination "PROMOIS WK-HQ" par la société SEIWA KASEI,
- les hydrolysats de kératine portant des groupements lauryl diméthyl ammonium tels que ceux vendus sous la dénomination "CROQUAT K" par la société CRODA,
- les hydrolysats de caséine portant des groupements N-hydroxypropyl triméthyl ammonium tels que ceux vendus sous la dénomination "MILKPRO Q" par la société SACI, ou des groupements lauryl diméthyl ammonium tels que ceux vendus sous la dénomination "HYDROLACTIN QL" par la société CRODA, ou des groupements triméthyl ammonium tels que ceux vendus sous la dénomination "HYDROLACTIN Q" par la société CRODA,
- les hydrolysats de fibroïne de soie portant des groupements cocoyl diméthyl N-hydroxypropyl ammonium tels que ceux vendus sous les dénominations "PROMOIS SILK-CAQ" par la société SEIWA KASEI ou "CROSILKQUAT par la société CRODA, ou des groupements lauryl diméthyl N-hydroxypropyl ammonium tels que ceux vendus sous la dénomination "PROMOIS SILK-LAQ" par la société SEIWA KASEI, ou des groupements stéaryl diméthyl N-hydroxypropyl ammonium tels que ceux vendus sous la dénomination "PROMOIS SILK-SAQ" par la société SEIWA KASEI,
- les hydrolysats de collagène portant des groupements N-hydroxypropyl triméthyl ammonium tels que ceux vendus sous la dénomination "CROMOIST CR" par la société CRODA, ou des groupements N-hydroxyéthyl triméthyl ammonium tels que ceux vendus sous la dénomination "CROTEINE Q" par la société CRODA, ou des groupements N-hydroxypropyl diméthyl laurylamidopropyl ammonium tels que ceux vendus sous la dénomination "MONTEINE LCQ" par la société SEPPIC, ou des groupements N-hydroxypropyl cocoyl diméthyl ammonium tels que ceux vendus sous la dénomination "LEXEIN QX 3000" par la société INOLEX, ou des groupements stéaryl triméthyl ammonium tels que ceux vendus sous la dénomination "QUAT-COLL QS" par la société QUIMDIS,
- les hydrolysats de protéines de blé portant des groupements stéaryl diméthyl N-hydroxypropyl ammonium tels que ceux vendus sous la dénomination "HYDROTITICUM QS" par la société CRODA, ou des groupements lauryl diméthyl N-hydroxypropyl ammonium tels que ceux vendus sous la dénomination "HYDROTITICUM QL" par la société CRODA, ou des groupements triméthyl N-hydroxypropyl ammonium tels que ceux vendus sous la dénomination "HYDROTITICUM WQ" par la société CRODA, ou des groupements cocoyl diméthyl N-hydroxypropyl ammonium tels que ceux vendus sous la dénomination "HYDROTITICUM QM" par la société CRODA,
- les hydrolysats de protéines de soja portant des groupements lauryl diméthyl N-hydroxypropyl ammonium tels que ceux vendus sous les dénominations "PROMOIS WS-LAQ" par la société SEIWA KASEI ou "CROQUAT SOYA" par la société CRODA, ou des groupements cocoyl diméthyl ammonium tels que ceux vendus sous la dénomination "PROMOIS WS-CAQ" par la société SEIWA KASEI, ou des groupements triméthyl N-hydroxypropyl ammonium tels que ceux vendus sous les dénominations "PROMOIS WS-Q" par la société SEIWA KASEI ou "PEPTEIN QS" par la société HORMEL.

Les dérivés de protéines peuvent être également des kératines à groupements sulfoniques telles que décrites dans le brevet FR-A-2 573 305. De telles kératines sont vendues sous la dénomination "OLIGO KERATINE" par la société CHEM Y ou sous la dénomination "MEXORYL SU" par la société CHIMEX.

On utilise de préférence, pour la mise en oeuvre du procédé selon l'invention, les dérivés protéiniques choisis parmi les hydrolysats de kératine, les hydrolysats de protéines de blé et les hydrolysats de protéines de soja, les hydrolysats pouvant être quaternisés ou non quaternisés.

Le poids moléculaire des protéines et de leurs dérivés utilisés selon l'invention est généralement compris entre 100 et 200 000, de préférence entre 400 et 100 000.

Selon la présente invention, on peut mettre en oeuvre les protéines et leurs dérivés seuls, mais le plus souvent on fait appel à des compositions contenant, dans un support cosmétiquement acceptable, de telles protéines. Elles peuvent ainsi être soit solubilisées, soit mises en dispersions ou en (micro)émulsions, dans des milieux aqueux ou des milieux organiques ou encore des milieux hydroorganiques. A titre de solvants organiques convenables, on peut notamment citer les monoalcools ou les polyols (éthanol, isopropanol, glycérol, alcool benzylique, glycols), l'acétone, les éthers de polyols, les hydrocarbures, le diméthoxyméthane, ou bien encore les silicones volatiles.

Les teneurs en protéines ou en dérivés protéiniques dans les compositions sont généralement comprises entre 0,1 % et 30 %, de préférence entre 0,5 % et 15 %, en poids par rapport au poids total de la composition.

Les compositions peuvent se présenter sous toute forme habituellement utilisée dans le domaine des compositions capillaires, telle que par exemple liquide plus ou moins épaissi ou gélifié, crème, mousse, lotion, gel, pâte, émulsion, spray, aérosol ou toute autre forme appropriée.

Les compositions à base de protéines peuvent ainsi, et d'une manière générale, contenir tous les divers additifs classiques qui sont utilisés dans le domaine de la préparation des compositions capillaires à usage topique et être choisis par exemple parmi des filtres UV, des agents épaississants, des agents de pénétration, des anti-oxydants, des agents sequestrants, des agents opacifiants, des tampons, des agents tensio-actifs choisis parmi les tensio-actifs non-ioniques tels que les alkylpolyglycosides, les tensio-actifs cationiques, les tensio-actifs anioniques et les tensio-actifs amphotères, des agents solubilisants, des émollients, des silicones, des colorants, des parfums et des conservateurs.

Des exemples concrets, mais nullement limitatifs, illustrant l'invention vont maintenant être donnés.

### Exemple 1

On a utilisé une composition 1 à base de protéines ayant les caractéristiques suivantes :

| | |
|---|---|
| - Hydrolysat de kératine de laine vendu sous la dénomination "PROMOIS WK-H" par la société SEIWA-KASEI | 5 g MA |
| - Amodiméthicone vendu sous la dénomination "DC 929" par la société DOW-CORNING | 0,2 g MA |
| - Parfum, colorant, conservateur | qs |
| - Eau déminéralisée qsp | 100 g |

Le mode opératoire est le suivant : on applique la composition ci-dessus sur des cheveux; puis on enroule les mèches ainsi traitées sur des bigoudis ; puis on traite pendant 3 secondes la mèche ainsi enroulée au moyen d'un jet de gaz ne contenant pour l'essentiel que de la vapeur d'eau et dont la température est de 90°C; la mèche ainsi traitée est ensuite refroidie au moyen d'un courant d'air ambiant, enfin on déroule la mèche du bigoudis.

Les cheveux ainsi traités présentent de belles boucles qui ont une très bonne tenue dans le temps.

### Exemple 2

On procède comme à l'exemple 1, à cette différence près qu'une composition 2 à base de protéines ayant les caractéristiques suivantes a été utilisée :

| | |
|---|---|
| - Hydrolysat de protéines de blé vendu sous la dénomination "CROPEPSOL W" par la société CRODA | 2 g MA |
| - Ethanol | 8,6 g |
| - Parfum, colorant, conservateur qs | |
| - Eau déminéralisée qsp | 100 g |

Les résultats obtenus sont comparables à ceux de l'exemple 1.

### Exemple 3

On procède comme à l'exemple 1, à cette différence près qu'une composition 3 à base de protéines ayant les caractéristiques suivantes a été utilisée :

| | |
|---|---|
| - Hydrolysat de kératine de laine quaternisé vendu sous la dénomination "PROMOIS WK-HLAQ" par la société SEIWA KASEI | 0,5 g MA |
| - Parfum, colorant, conservateur qs | |
| - Eau déminéralisée qsp | 100 g |

Les résultats obtenus sont comparables à ceux de l'exemple 1.

### Exemple 4

On procède comme à l'exemple 1, à cette différence près qu'une composition 4 à base de protéines ayant les caractéristiques suivantes a été utilisée :

| | |
|---|---|
| - Kératine sulfonée vendu sous la dénomination "OLIGO KERATIN" par la société CHEM Y | 1 g MA |
| - Parfum, colorant, conservateur qs | |
| - Eau déminéralisée qsp | 100 g |

Les résultats obtenus sont comparables à ceux de l'exemple 1.

### Exemple 5

On procède comme à l'exemple 1, à cette différence près qu'une composition 5 à base de protéines ayant les caractéristiques suivantes a été utilisée :

| | |
|---|---|
| - Hydrolysat de protéines de soja quaternisé vendu sous la dénomination "CROQUAT SOYA" par la société CRODA | 10 g MA |
| - Amodiméthicone vendu sous la dénomination "DC 929" par la société DOW CORNING | 1 g MA |
| - Parfum, colorant, conservateur | qs |
| - Eau déminéralisée qsp | 100 g |

Les résultats obtenus sont comparables à ceux de l'exemple 1.

### Exemple 6 (comparatif)

On a utilisé des mèches de cheveux de 20 cm de long que l'on a traitées par immersion dans une solution de protéines à 5 % de matière active, à 50°C. Après essorage et enroulage sur bigoudi de 20 mm de diamètre, la mèche a été séchée sous casque, puis traitée par de la vapeur d'eau à 100 °C pendant 45 secondes, repassée sous casque jusqu'à séchage complet, puis laissée au dessicateur.

En parallèle, on a effectué sur d'autres mèches le même mode opératoire mais sans traitement à la vapeur.

On a déterminé sur les mèches ainsi traitées la tenue à la déformation et la tenue au démêlage.

Une comparaison a également été effectuée par rapport à des mèches humidifiées à l'eau, sans être traitées avec une solution de protéines.

### Tenue à la déformation:

On a déroulé la mèche traitée, on a donné deux coups de peigne, on a allongé la mèche en la tirant sur ces deux extrémités, puis on l'a posée bien à plat sur un support horizontal et enfin, on a posé sur celle-ci, toujours à plat, un poids d'environ 15 kg qui a été laissé pendant 4 heures.
On a mesuré la longueur de la mèche avant de poser le poids (tₒ) et après les 4 heures de contrainte (t). On a déterminé la variation de la longueur de la mèche entre t et tₒ.

On a obtenu les résultats suivants :

| **Solution de traitement** | **Traitement à la vapeur** | **Variation de la longueur de mèche** |
|---|---|---|
| Eau | NON | + 5,5 cm |
| Eau | OUI | + 3 cm |
| KERASOL (1) | NON | + 5 cm |
| KERASOL (1) | OUI | + 1,8 cm |
| PROMOIS WK-HLAQ (2) | NON | + 4,9 cm |
| PROMOIS WK-HLAQ (2) | OUI | + 1,3 cm |

| | | |
|---|---|---|
| (1) KERASOL : hydrolysat de kératine de sabot vendu par la société CRODA | | |
| (2) PROMOIS WK-HLAQ : hydrolysat de kératine de laine quaternisé vendu par la société SEIWA-KASEI | | |

On constate que les variations de longueur de mèches sont beaucoup plus faibles pour les mèches traitées avec une solution de protéines et avec la vapeur; ces mèches présentent donc la meilleur tenue à la déformation.

### Tenue au démêlage :

On a déroulé la mèche traitée, on l'a suspendue à la verticale et on a mesuré la longueur de la mèche ainsi soumise à son propre poids. Puis on a effectué 80 coups de peigne et on a mesuré à nouveau la longueur de la mèche après peignage. On détermine ensuite la variation de la longueur de la mèche due au coups de peigne.

On a obtenu les résultats suivants :

| **Solution de traitement** | **Traitement à la vapeur** | **Variation de la longueur de mèche** |
|---|---|---|
| Eau | NON | + 3 cm |
| Eau | OUI | + 4 cm |
| PROMOIS WK-H (3) | NON | + 3 cm |
| PROMOIS WK-H (3) | OUI | + 2 cm |

| | | |
|---|---|---|
| (3) PROMOIS WK-H : hydrolysat de kératine de laine vendu par la société SEIWA KASEI | | |

On constate que les variations de longueur de mèches sont plus faibles pour les mèches traitées avec une solution de protéines et avec la vapeur ; ces mèches présentent donc la meilleur tenue au peignage.

## Revendications

1. Procédé de traitement pour la déformation non permanente des fibres kératiniques humaines, en particulier des cheveux, caractérisé par le fait qu'il consiste (i) à mettre en contact un gaz contenant de la vapeur d'eau et dont la température est d'au moins 75°C, avec des fibres kératiniques humaines maintenues sous tension mécanique et sur lesquelles on a appliqué une composition contenant au moins une protéine et/ou un dérivé protéinique, (ii) à refroidir les fibres ainsi traitées et enfin (iii) à supprimer la tension mécanique qui était appliquée sur les fibres.

2. Procédé selon la revendication 1, caractérisé par le fait que ledit gaz a une température supérieure ou égale à 85°C.

3. Procédé selon la revendication 2, caractérisé par le fait que ladite température est comprise entre 85°C et 150°C.

4. Procédé selon l'une quelconque des revendications précédentes, caractérisé par le fait que ledit gaz est mis en contact avec la fibre à déformer pendant une durée allant de 0,01 seconde à 2 minutes.

5. Procédé selon la revendication 4, caractérisé par le fait que ladite durée est comprise entre 0,01 seconde et 30 secondes.

6. Procédé selon la revendication 5, caractérisé par le fait que ladite durée est comprise entre 1 seconde et 10 secondes.

7. Procédé selon l'une quelconque des revendications précédentes, caractérisé par le fait que l'application du gaz est répétée plusieurs fois sur une même fibre.

8. Procédé selon l'une quelconque des revendications précédentes, caractérisé par le fait que ledit gaz ne contient que de la vapeur d'eau.

9. Procédé selon l'une quelconque des revendications 1 à 7, caractérisé par le fait que ledit gaz contient de la vapeur d'eau et au moins un autre composé sous forme de gaz ou de vapeur.

10. Procédé selon la revendication 9, caractérisé par le fait que ledit gaz contient de la vapeur d'eau et de l'air.

11. Procédé selon l'une quelconque des revendications précédentes, caractérisé par le fait que la protéine est choisie parmi les protéines issues de matières d'origine animale ou végétale.

12. Procédé selon l'une quelconque des revendications précédentes, caractérisé par le fait que la protéine est issue de matière d'origine animale et est choisie parmi la kératine, l'élastine, le collagène, les protéines du lait, l'albumine de blanc d'oeuf.

13. Procédé selon l'une quelconque des revendications 1 à 11, caractérisé par le fait que la protéine est issue de matière d'origine végétale et est choisie parmi les protéines du blé, de germes de blé, de l'avoine, de l'orge, du maïs, du riz, du soja, de fèves, de graines de coton, de graines de lupin, de pommes de terre, de noyaux d'abricots.

14. Procédé selon l'une quelconque des revendications précédentes, caractérisé par le fait que le dérivé protéinique est un hydrolysat de protéines.

15. Procédé selon la revendication 14, caractérisé par le fait que l'hydrolysat de protéines est choisi parmi les hydrolysats de protéines d'algues, d'élastine, de blé, de soja, de lupin, de kératine, de caséine, de lactosérum, de blanc d'oeuf, de soie.

16. Procédé selon les revendications 14 ou 15, caractérisé par le fait que l'hydrolysat de protéines est quaternisé.

17. Procédé selon l'une quelconque des revendications 1 à 12, caractérisé par le fait que le dérivé protéinique est une kératine à groupements sulfoniques.

18. Procédé selon l'une quelconque des revendications précédentes, caractérisé par le fait que les protéines et/ou leurs dérivés protéiniques sont présents dans ladite composition à une teneur comprise entre 0,1 et 30 % en poids par rapport au poids total de la composition.

## Claims

1. Treatment process for the temporary reshaping of human keratin fibres, in particular the hair, characterized in that it consists (i) in placing a gas containing steam and whose temperature is at least 75°C, in contact with human keratin fibres maintained under mechanical tension and on which a composition containing at least one protein and/or one protein derivative has been applied, (ii) in cooling the fibres thus treated and, finally, (iii) in removing the mechanical tension which was applied to the fibres.

2. Process according to Claim 1, characterized in that the said gas has a temperature above or equal to 85°C.

3. Process according to Claim 2, characterized in that the said temperature is between 85°C and 150°C.

4. Process according to any one of the preceding claims, characterized in that the said gas is placed in contact with the fibre to be reshaped for a period ranging from 0.01 second to 2 minutes.

5. Process according to Claim 4, characterized in that the said period is between 0.01 second and 30 seconds.

6. Process according to Claim 5, characterized in that the said period is between 1 second and 10 seconds.

7. Process according to any one of the preceding claims, characterized in that application of the gas is repeated several times on the same fibre.

8. Process according to any one of the preceding claims, characterized in that the said gas contains only steam.

9. Process according to any one of Claims 1 to 7, characterized in that the said gas contains steam and at least one other compound in the form of gas or vapour.

10. Process according to Claim 9, characterized in that the said gas contains steam and air.

11. Process according to any one of the preceding claims, characterized in that the protein is chosen from proteins derived from materials of animal or plant origin.

12. Process according to any one of the preceding claims, characterized in that the protein is derived from material of animal origin and is chosen from keratin, elastin, collagen, milk proteins and albumin from egg white.

13. Process according to any one of Claims 1 to 11, characterized in that the protein is derived from material of plant origin and is chosen from proteins from wheat, from wheatgerm, from oats, from barley, from corn, from rice, from soya, from beans, from cotton seeds, from lupin seeds, from potatoes and from apricot kernels.

14. Process according to any one of the preceding claims, characterized in that the protein derivative is a protein hydrolysate.

15. Process according to Claim 14, characterized in that the protein hydrolysate is chosen from the protein hydrolysates of algae, of elastin, of wheat, of soya, of lupin, of keratin, of casein, of lactoserum, of egg white and of silk.

16. Process according to Claims 14 or 15, characterized in that the protein hydrolysate is quaternized.

17. Process according to any one of Claims 1 to 12, characterized in that the protein derivative is a keratin containing sulphonic groups.

18. Process according to any one of the preceding claims, characterized in that the proteins and/or the protein derivatives are present in the said composition at a content between 0.1 and 30 % by weight relative to the total weight of the composition.

## Patentansprüche

1. Verfahren zur Behandlung von menschlichen Keratinfasern, insbesondere dem Haar, zur nicht dauerhaften Verformung,
dadurch gekennzeichnet, daß
es aus folgenden Schritten besteht:
(i) Inkontaktbringen eines Gases, das Wasserdampf enthält und dessen Temperatur mindestens 75 °C beträgt, mit menschlichen Keratinfasern, die unter mechanischer Spannung gehalten werden und auf die eine Zusammensetzung aufgebracht wurde, die mindestens ein Protein und/oder ein Proteinderivat enthält,
(ii) Abkühlen der so behandelten Fasern, und schließlich
(iii) Aufheben der mechanischen Spannung, die auf die Fasern ausgeübt worden ist.

2. Verfahren nach Anspruch 1, dadurch gekennzeichnet, daß das Gas eine Temperatur von mindestens 85 °C aufweist.

3. Verfahren nach Anspruch 2, dadurch gekennzeichnet, daß die Temperatur im Bereich von 85 bis 150 °C liegt.

4. Verfahren nach einem der vorhergehenden Ansprüche, dadurch gekennzeichnet, daß das Gas mit den zu verformenden Fasern während einer Dauer von 0,01 s bis 2 min in Kontakt gebracht wird.

5. Verfahren nach Anspruch 4, dadurch gekennzeichnet, daß die Dauer 0,01 s bis 30 s beträgt.

6. Verfahren nach Anspruch 5, dadurch gekennzeichnet, daß die Dauer 1 s bis 10 s beträgt.

7. Verfahren nach einem der vorhergehenden Ansprüche, dadurch gekennzeichnet, daß man das Gas mehrmals auf die gleichen Fasern einwirken läßt.

8. Verfahren nach einem der vorhergehenden Ansprüche, dadurch gekennzeichnet, daß das Gas ausschließlich Wasserdampf enthält.

9. Verfahren nach einem der vorhergehenden Ansprüche 1 bis 7, dadurch gekennzeichnet, daß das Gas Wasserdampf und mindestens eine weiter Verbindung in Form von Gas oder Dampf enthält.

10. Verfahren nach Anspruch 9, dadurch gekennzeichnet, daß das Gas Wasserdampf und Luft enthält.

11. Verfahren nach einem der vorhergehenden Ansprüche, dadurch gekennzeichnet, daß das Protein unter Proteinen ausgewählt ist, die von Materialien tierischen oder pflanzlichen Ursprungs stammen.

12. Verfahren nach einem der vorhergehenden Ansprüche, dadurch gekennzeichnet, daß das Protein von Materialien tierischen Ursprungs stammt und ausgewählt ist unter Keratin, Elastin, Kollagen, Milchproteinen und Albumin von Eiklar.

13. Verfahren nach einem der Ansprüche 1 bis 11, dadurch gekennzeichnet, daß das Protein von Materialien pflanzlichen Ursprungs stammt und ausgewählt ist unter Proteinen von Weizen, Weizenkeimen, Hafer, Gerste, Mais, Reis, Soja, dicken Bohnen, Baumwollsamen, Lupinensamen, Kartoffeln und Aprikosenkernen.

14. Verfahren nach einem der vorhergehenden Ansprüche, dadurch gekennzeichnet, daß das Proteinderivat ein Proteinhydrolysat ist.

15. Verfahren nach Anspruch 14, dadurch gekennzeichnet, daß das Proteinhydrolysat ausgewählt ist unter Hydrolysaten von Proteinen aus Algen, Elastin, Weizen, Soja, Lupinen, Keratin, Casein, Molke, Eiklar und Seide.

16. Verfahren nach Anspruch 14 oder 15, dadurch gekennzeichnet, daß das Proteinhydrolysat quaternisiert ist.

17. Verfahren nach einem der Ansprüche 1 bis 12, dadurch gekennzeichnet, daß das Proteinderivat ein Keratin mit Sulfongruppen ist.

18. Verfahren nach einem der vorhergehenden Ansprüche, dadurch gekennzeichnet, daß die Proteine und/oder ihre Proteinderivate in der Zusammensetzung in Anteilen von 0,1 bis 30 Gew.-%, bezogen auf das Gesamtgewicht der Zusammensetzung, vorliegen.
